(19) Europäisches Patentamt
European Patent Office
Office européen des brevets

(11) Veröffentlichungsnummer : **0 019 578**
**B1**

(12) # EUROPÄISCHE PATENTSCHRIFT

(45) Veröffentlichungstag der Patentschrift : **30.03.83**

(21) Anmeldenummer : **80810117.4**

(22) Anmeldetag : **03.04.80**

(51) Int. Cl.³ : **C 07 D401/14**, C 07 D211/58, C 08 K 5/34, C 08 L 23/00 // (C07D401/14, 241/04, 211/58),(C07D401/14, 241/08, 211/58),(C07D401/14, 243/08, 211/58)

(54) **Piperidinverbindungen, Verfahren zu deren Herstellung und diese enthaltende polymere Zusammensetzungen.**

(30) Priorität : **13.04.79 IT 2184179**

(43) Veröffentlichungstag der Anmeldung : **26.11.80 Patentblatt 80/24**

(45) Bekanntmachung des Hinweises auf die Patenterteilung : **30.03.83 Patentblatt 83/13**

(84) Benannte Vertragsstaaten : **DE FR GB IT**

(56) Entgegenhaltungen :
**DE A 2 118 298**
**DE A 2 314 115**
**FR A 2 398 750**
**GB A 1 392 249**
**GB A 1 529 903**

(73) Patentinhaber : **Chimosa Chimica Organica S.p.A.**
**Via Pila 6/3**
**I-40044 Pontecchio Marconi (Bologna) (IT)**

(72) Erfinder : **Cantatore, Giuseppe**
**Via Lido 112**
**Casalecchio de Reno (Bologna) (IT)**
Erfinder : **Cassandrini, Paolo**
**Via Nino Scota 11**
**Bologna (IT)**

(74) Vertreter : **Stamm, Otto et al**
**CIBA-GEIGY AG Postfach**
**CH-4002 Basel (CH)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 019 578 B1

# Piperidinverbindungen, Verfahren zu deren Herstellung und diese enthaltende polymere Zusammensetzungen

Die Erfindung betrifft neue, als Licht-, Wärme- und Oxydationsstabilisatoren für synthetische Polymere verwendbare Piperidinverbindungen, ein Verfahren zu deren Herstellung sowie diese enthaltende polymere Zusammensetzungen.

Im einzelnen betrifft die Erfindung neue Verbindungen der Formel I

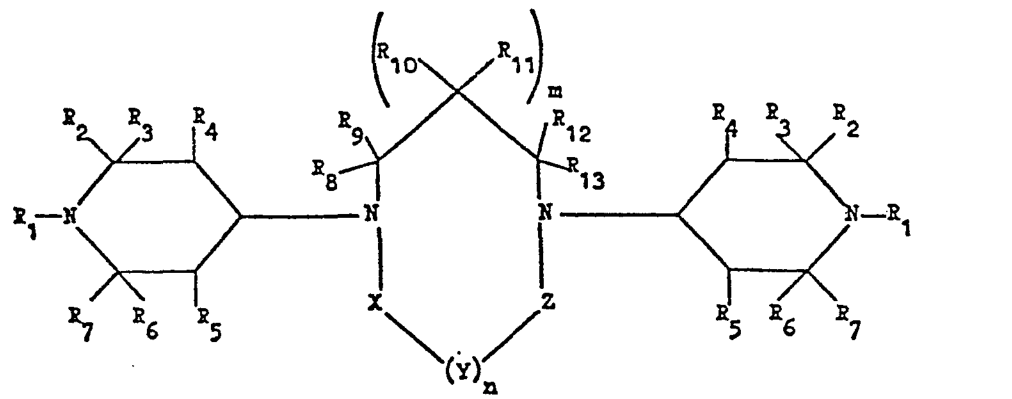

(I)

worin $R_1$ Wasserstoff, —O·, —CN, geradkettiges oder verzweigtes Alkyl mit 1 bis 20 Kohlenstoffatomen, Alkenyl oder Alkinyl mit 2 bis 20 Kohlenstoffatomen, gegebenenfalls durch $C_1$-$C_4$-Alkyl einfach bis dreifach substituiertes Benzyl oder Hydroxybenzyl, einen Rest $—COR_{14}$, $-COOR_{14}$, $—CH_2COOR_{14}$ oder $—CONR_{14}R_{15}$, worin $R_{14}$ und $R_{15}$ gleich oder verschieden sein können und für geradkettiges oder verzweigtes $C_1$-$C_{20}$-Alkyl, $C_2$-$C_{20}$-Alkenyl, $C_5$-$C_{12}$-Cycloalkyl, gegebenenfalls durch $C_1$-$C_8$-Alkyl einfach bis dreifach substituiertes Phenyl, gegebenenfalls durch $C_1$-$C_4$-Alkyl einfach bis dreifach substituiertes Hydroxyphenyl, $C_7$-$C_{12}$-Aralkyl, 2,2,6,6-Tetramethyl-4-piperidyl, 1,2,2,6,6-Pentamethyl-4-piperidyl stehen oder, wenn sie an N gebunden sind, Wasserstoff sein oder zusammen mit dem N, an das sie gebunden sind, einen stickstoffhaltigen 5- bis 7-gliedrigen heterocyclischen Ring bilden können, einen Rest $—CH_2—\underset{\displaystyle R_{16}}{\underset{|}{C}H}—R_{17}$, worin $R_{16}$ für Wasserstoff oder Methyl und $R_{17}$ für $—OH$, $—OR_{14}$, $—OCOR_{14}$ oder $—OCONR_{14}R_{15}$ stehen, worin $R_{14}$ und $R_{15}$ die oben angegebene Bedeutung haben, oder einen Rest

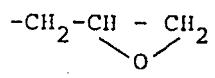

darstellt, $R_2$, $R_3$, $R_6$ und $R_7$ gleich oder verschieden sein können und Alkyl mit 1 bis 6 C-Atomen bedeuten, $R_4$ und $R_5$ gleich oder verschieden sein können und für Wasserstoff oder Alkyl mit 1 bis 6 C-Atomen stehen, $R_8$, $R_9$, $R_{10}$, $R_{11}$, $R_{12}$ und $R_{13}$ gleich oder verschieden sein können und Wasserstoff oder Alkyl mit 1 bis 6 C-Atomen darstellen, m und n null oder 1 sind, X und Z gleich oder verschieden sein können und

$>C=O$ oder 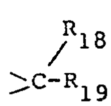

bedeuten, worin $R_{18}$ für Wasserstoff oder $C_1$-$C_{20}$-Alkyl und $R_{19}$ für Wasserstoff, $C_1$-$C_{20}$-Alkyl oder einen Rest $—(CH_2)_r—COOR_{14}$ oder $—CONR_{14}R_{15}$ stehen, wobei $R_{14}$ und $R_{15}$ die oben angegebene Bedeutung haben und r eine ganze Zahl von 0 bis 10 ist, sowie

Y $>C=O$ oder 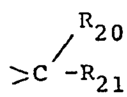

darstellt, worin $R_{20}$ für Wasserstoff oder $C_1$-$C_{20}$-Alkyl und $R_{21}$ für Wasserstoff, $C_1$-$C_{20}$-Alkyl, gegebenenfalls durch $C_1$-$C_4$-Alkyl einfach bis dreifach substituiertes Benzyl oder Hydroxybenzyl, 2,2,6,6-Tetramethyl-4-piperidyl, 1,2,2,6,6-Pentamethyl-4-piperidyl oder einen Rest —OH, $—NO_2$, $—NR_{22}R_{23}$ oder $—NH—COR_{24}$ stehen, wobei $R_{22}$ und $R_{23}$ gleich oder verschieden sein können und Wasserstoff, $C_1$-$C_{20}$-Alkyl, Benzyl oder gegebenenfalls durch $C_1$-$C_4$-Alkyl einfach bis dreifach substituiertes Hydroxybenzyl sowie $R_{24}$ $C_1$-$C_{20}$-Alkyl, gegebenenfalls durch $C_1$-$C_8$-Alkyl einfach bis dreifach substituiertes Phenyl oder durch $C_1$-$C_4$-Alkyl einfach bis dreifach substituiertes Hydroxyphenyl bedeuten.

Als spezielle Beispiele zur Veranschaulichung der Bedeutungen der verschiedenen Reste seien die folgenden angegeben :

für $R_1$ : Wasserstoff, —O·, —CN, Methyl, Aethyl, n-Propyl, n-Butyl, Isobutyl, n-Hexyl, n-Octyl, n-Decyl, n-Dodecyl, n-Octadecyl, Allyl, 2-Butenyl, 10-Undecenyl, Oleyl, Propargyl, Benzyl, 4-Methylbenzyl, 4-t-Butylbenzyl, 4-Hydroxybenzyl und 3,5-Di-t-butyl-4-hydroxybenzyl ;

für $R_{14}$ und $R_{15}$ : Methyl, Aethyl, n-Propyl, n-Butyl, n-Hexyl, n-Octyl, n-Decyl, n-Dodecyl, Allyl, 2-Butenyl, 10-Undecenyl, Oleyl, Cyclohexyl, 2- oder 4-Methylcyclohexyl, 3,3,5-Trimethylcyclohexyl, Phenyl, 2- oder 4-Methylphenyl, 2,4- oder 2,6-Dimethylphenyl, 4-t-Butylphenyl, 3,5-Di-t-butyl-4-hydroxyphenyl, Benzyl, 4-Methylbenzyl, 4-t-Butylbenzyl, 2,2,6,6-Tetramethyl-4-piperidyl und 1,2,2,6,6-Pentamethyl-4-piperidyl ; ferner, falls $R_{14}$ und $R_{15}$ an ein N gebunden sind, so sind sie vorzugsweise Wasserstoff oder bilden zusammen mit dem N-Atom, an das sie gebunden sind, beispielsweise einen Teil eines Pyrrolidin-, Piperidin-, Morpholin-, Piperazin-, N-Methylpiperazin-, Homopiperazin- oder N-Methylhomopiperazinrings ; typische Beispiele der Gruppe $—CH_2—\underset{R_{16}}{\underset{|}{C}}H—R_{17}$ sind : $—CH_2CH_2OH$ ; $—CH_2CHOHCH_3$, $—CH_2CH_2OCH_3$, $—CH_2CH_2OC_2H_5$, $—CH_2CH_2OC_4H_9$, $—CH_2CH_2OCOCH_3$, $—CH_2CH_2OCOC_3H_7$, $—CH_2CH_2O-CON(CH_3)_2$, $—CH_2CH_2OCON(C_2H_5)_2$ ;

für $R_2$, $R_3$, $R_6$ und $R_7$ : Methyl und Aethyl ;

für $R_4$, $R_5$, $R_8$, $R_9$, $R_{10}$, $R_{11}$, $R_{12}$ und $R_{13}$ : Wasserstoff und Methyl ;

für $R_{18}$, $R_{19}$, $R_{20}$, $R_{22}$ und $R_{23}$ : Wasserstoff, Methyl, Aethyl, n-Butyl, Isobutyl, n-Hexyl, n-Octyl, n-Decyl, n-Dodecyl, n-Hexadecyl und n-Octadecyl ;

für $R_{21}$ : Wasserstoff, Methyl, Aethyl, n-Butyl, n-Hexyl, n-Octyl, n-Decyl, n-Dodecyl, n-Hexadecyl, n-Octadecyl, Benzyl, 4-Methylbenzyl, 4-t-Butylbenzyl, 4-Hydroxybenzyl, 3,5-Di-t-butyl-4-hydroxybenzyl, —OH und $—NO_2$ ;

für $R_{24}$ : Methyl, Aethyl, n-Butyl, Isobutyl, n-Hexyl, n-Octyl, n-Decyl, n-Dodecyl, n-Hexadecyl, n-Octadecyl, Phenyl, 2- oder 4-Methylphenyl, 4-t-Butylphenyl und 3,5-Di-t-butyl-4-hydroxyphenyl.

Solche Verbindungen der Formel I werden bevorzugt, worin $R_1$ Wasserstoff oder $C_1$-$C_6$-Alkyl darstellt, $R_2$, $R_3$, $R_6$ und $R_7$ Methyl oder Aethyl bedeuten, $R_4$, $R_5$, $R_8$, $R_9$, $R_{10}$, $R_{11}$, $R_{12}$ und $R_{13}$ für Wasserstoff oder Methyl stehen,

$$X \text{ und } Z >C=O \text{ oder } >C{\overset{R_{18}}{\diagup}}-R_{19}$$

darstellen, wobei $R_{18}$ vorzugsweise Wasserstoff oder Methyl sowie $R_{19}$ Wasserstoff oder Aethoxycarbonyl bedeuten,

$$Y >C{\overset{R_{20}}{\diagup}}{\underset{R_{21}}{\diagdown}}$$

darstellt, worin $R_{20}$ vorzugsweise für Wasserstoff oder $C_1$-$C_{12}$-Alkyl und $R_{21}$ für Wasserstoff, $C_1$-$C_{12}$-Alkyl, Benzyl, 3,5-Di-t-butyl-4-hydroxybenzyl, —OH, $—NO_2$ oder $—NH_2$ stehen, sowie m und n null oder 1 sind.

Solche Verbindungen der Formel I werden bevorzugt, worin $R_2$ und $R_6$ für Aethyl, $R_3$, $R_4$ und $R_7$ für Methyl sowie $R_5$ für Wasserstoff stehen.

Besonders bevorzugt werden solche Verbindungen der Formel I, worin $R_2$, $R_3$, $R_6$ und $R_7$ für Methyl sowie $R_4$ und $R_5$ für Wasserstoff stehen.

Die neuen erfindungsgemässen Piperidinverbindungen lassen sich durch Cyclisierung von N,N'-Bis-(polyalkyl-4-piperidyl)-alkylendiaminen der Formel II

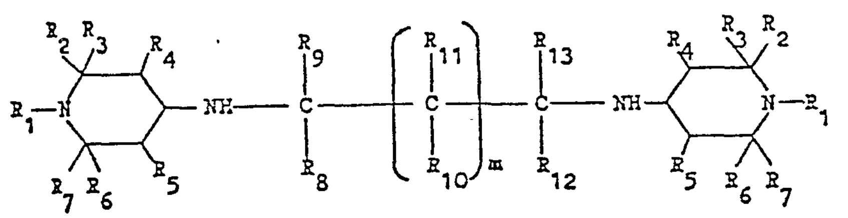

(II)

worin $R_1$, $R_2$, $R_3$, $R_4$, $R_5$, $R_6$, $R_7$, $R_8$, $R_9$, $R_{10}$, $R_{11}$, $R_{12}$ und $R_{13}$ die oben angegebe Bedeutung haben

und m null oder 1 ist, herstellen.

Die Cyclisierung erfolgt im allgemeinen durch Umsetzung mit Verbindungen der Formel III

$$A—X—(Y)_n—Z—B \quad (III)$$

worin X, Y, Z und n die bereits angegebene Bedeutung haben sowie A und B für Halogen, vorzugsweise Chlor oder Brom stehen, bzw., nur wenn X und Z >C=O sind, für eine Gruppe $—OR_{25}$ stehen können, worin $R_{25}$ $C_1$-$C_4$-Alkyl, Phenyl oder Methylphenyl bedeutet.

Im Fall, wo

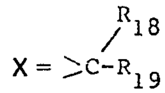

mit $R_{18}$ und $R_{19}$=H oder Alkyl und Z=>C=O, können die Verbindungen der Formel I auch durch Cyclisierung von Verbindungen der Formel II durch Umsetzung mit Verbindungen der Formel IV

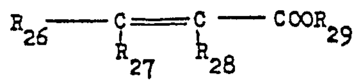

(IV)

worin $R_{26}$, $R_{27}$ und $R_{28}$ gleich oder verschieden sein können und für Wasserstoff oder $C_1$-$C_4$-Alkyl stehen und $R_{29}$ vorzugsweise $C_1$-$C_4$-Alkyl, Phenyl oder Methylphenyl bedeutet, hergestellt werden.

Schliesslich kann man im Sonderfall, wo

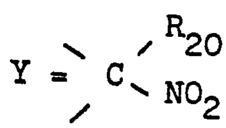

die Verbindungen der Formel I auch durch Cyclisierung von Verbindungen der Formel II mit Formaldehyd und Nitroverbindungen der Formel V

$$R_{20}—CH_2—NO_2 \quad (V)$$

worin $R_{20}$ die oben angegebene Bedeutung hat, herstellen.

Die so erhaltenen Nitroverbindungen werden vorzugsweise anschliessend einer Reduktion der $NO_2$-Gruppe zur $NH_2$-Gruppe unterworfen ; eine solche Reduktion kann nach verschiedenen bereits bekannten Methoden, beispielsweise mit Wasserstoff in Gegenwart von Katalysatoren wie Platin, Palladium und ähnlichen, erfolgen.

Bevorzugte Beispiele für bei der Herstellung von Produkten der Formel I verwendbare Verbindungen der Formel III sind : 1,2-Dichloräthan, 1,2-Dibromäthan, 1,2-Dibrompropan, 1,2-Dibrombutan, 1,2-Dibromhexan, 1,2-Dibromoctan, 1,3-Dichlor-2-propanol, Epichlorhydrin, Aethyl-2,3-dibrompropionat, Aethyl-2,3-dibromsuccinat, Aethyl-3,4-dibrombutyrat, Aethyl-10,11-dibromundecanoat, Aethyl-9,10-dibromstearat, Methyl-chloracetat, Aethyl-bromacetat, Methyl-2-brompropionat, Methyl-2-brombutyrat, Methyl-2-bromcaproat, Dimethyl- oder Diäthyloxalat, Dimethyl- oder Diäthylmalonat, Dimethyl-äthylmalonat, Dimethyl-diäthylmalonat, Dimethyl-n-butylmalonat, Dimethyl-isobutylmalonat, Dimethyl-n-hexylmalonat, Dimethyl-n-octylmalonat, Dimethyl-n-dodecylmalonat, Dimethylbenzylmalonat und Dimethyl-3,5-di-t-butyl-4-hydroxybenzylmalonat.

Beispiele für Verbindungen der Formel IV sind : Methyl- oder Aethylacrylat, Methylmethacrylat, Methyl-3,3-dimethylacrylat und methylcrotonat.

Beispiele für bei der Herstellung von Verbindungen der Formel I verwendbare Verbindungen der Formel V sind : Nitroäthan, 1-Nitropropan, 1-Nitrobutan, 1-Nitrohexan, 1-Nitrooctan, 1-Nitrodecan, 1-Nitrododecan und 1-Nitrooctadecan.

Die Verbindungen der Formel I, in denen $R_1$ von Wasserstoff verschieden ist, sind dadurch erhältlich, dass man von schon an den Piperidin-NH substituierten Dipiperidyldiaminen der Formel II ausgeht oder an den NH-Gruppen der Verbindungen der Formel I, worin $R_1$ Wasserstoff ist, eine Substitution vornimmt.

Dabei kann man Verbindungen der Formel I, in denen $R_1$ ein Oxylrest ist, aus den entsprechenden Verbindungen, in denen $R_1$ Wasserstoff ist, durch Umsetzung mit Wasserstoffperoxid in Gegenwart von Natriumwolframat oder durch Umsetzung mit einer Persäure, beispielsweise m-Chlorperbenzoesäure, erhalten ; Verbindungen, in denen $R_1$ eine —CN-Gruppe ist, sind aus den entsprechenden Verbindungen, in denen $R_1$ Wasserstoff ist, durch Umsetzung mit CNCl und CNBr erhältlich ; Verbindungen, in denen $R_1$ eine Alkyl-, Alkenyl-, Alkinyl- oder Benzylgruppe ist, können aus den entsprechenden Verbindungen, in denen $R_1$ Wasserstoff ist, durch Umsetzung mit Alkyl-, Alkenyl-, Alkinyl- oder gegebenenfalls substituierten Benzylhalogeniden hergestellt werden ; Verbindungen, in denen $R_1$ Methyl ist, sind auch aus

entsprechenden Verbindungen, in denen $R_1$ Wasserstoff ist, durch Umsetzung mit Formaldehyd und Ameisensäure herstellbar (Eschweiler-Clarke-Reaktion, Organic Reactions, Band V, Seite 307, Wiley & Sons, 1962); Verbindungen, in denen $R_1$ für eine Gruppe $-COR_{14}$, $-COOR_{14}$, $-CH_2COOR_{14}$ oder $-CONR_{14}R_{15}$ steht, lassen sich aus entsprechenden Verbindungen, in denen $R_1$ Wasserstoff ist, durch Umsetzung mit Halogenverbindungen des Typs $E-COR_{14}$, $E-COOR_{14}$, $E-CH_2COOR_{14}$ oder $E-CONR_{14}R_{15}$, in denen E für Halogen, vorzugsweise Chlor, Brom oder Jod steht sowie $R_{14}$ und $R_{15}$ die zuvor angegebene Bedeutung haben, herstellen; Verbindungen, in denen $R_1-CH_2-\underset{R_{16}}{\underset{|}{CH}}-R_{17}$ darstellt, kann man aus entsprechenden Verbindungen, in denen $R_1$ Wasserstoff ist, durch Umsetzung mit Aethylenoxyd oder Propylenoxyd, falls $R_{17}$ OH ist, bzw., falls $R_{17}-OR_{14}$, $-OCOR_{14}$ oder $-O-CONR_{14}R_{15}$ ist, durch anschliessende Umsetzung der hydroxylierten Verbindung mit einer Halogenverbindung des Typs $E-R_{14}$, $E-COR_{14}$ oder $E-CONR_{14}R_{15}$, worin E, $R_{14}$ und $R_{15}$ die oben angegebene Bedeutung haben, herstellen; die Verbindungen, in denen $R_1$ eine 2,3-Epoxypropylgruppe ist, sind durch Umsetzung der entsprechenden Verbindungen, in denen $R_1$ Wasserstoff ist, mit Epichlorhydrin und Aetzalkali erhältlich.

Die Verbindungen der Formel II, welche die Ausgangsstoffe für das erfindungsgemässe Verfahren darstellen, erhält man durch reduktive Substitution, in Gegenwart von Wasserstoff oder einem Hydrierungskatalysator von Alkylendiaminen der Formel VI

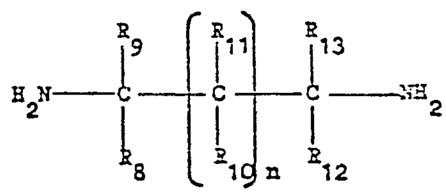

(VI)

mit Polyalkyl-4-piperidonen der Formel VII

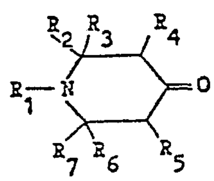

(VII)

wie in der US-Patentschrift 3 480 635 beschrieben.

Zur besseren Veranschaulichung der Erfindung seien im folgenden einige Beispiele für die Herstellung von Verbindungen der Formel I angegeben, wobei es sich versteht, dass solche Beispiele ausschliesslich der Erläuterung dienen und keine Beschränkung darstellen.

Beispiel 1

Man erhitzt 338 g (1 Mol) N,N'-Bis-(2,2,6,6-tetramethyl-4-piperidyl)-äthylendiamin, 206,6 g (1,1 Mol) 1,2-Dibromäthan, 276,4 g (2 Mol) wasserfreies Kaliumcarbonat und 2 000 ml Toluol 30 Stunden unter Rückfluss.

Das Reaktionsgemisch wird heiss filtriert, eingeengt und abgekühlt.

Den erhaltenen kristallinen Niederschlag trennt man durch Filtrieren ab und kristallisiert aus Toluol um.

So erhält man die Verbindung der Formel

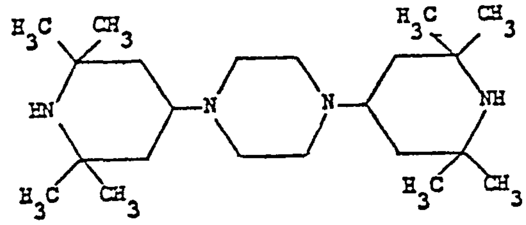

weisse Kristalle, Schmelzpunkt 198-9 °C.

Analyse für $C_{22}H_{44}N_4$
berechnet: C 72,47 %; H 12,16 %; N 15,36 %
gefunden: C 71,78 %; H 11,98 %; N 15,28 %.

Beispiel 2

Man erhitzt 72,9 g (0,2 Mol) gemäss Beispiel 1 hergestelltes N,N'-Bis-(2,2,6,6-tetramethyl-4-piperidyl)-piperazin, 35,7 g (0,66 Mol) 85 %ige Ameisensäure und 59,2 g (0,73 Mol) 37 %igen Formaldehyd 10 Stunden unter Rückfluss.

Nach Abkühlung wird das Reaktionsgemisch mit einer Lösung von 20 g Natriumhydroxyd in 200 ml Wasser zersetzt ; den erhaltenen Niederschlag trennt man durch Filtrieren ab, wäscht mit Wasser bis zu neutralem pH, trocknet und kristallisiert aus Toluolum.

So erhält man die Verbindung der Formel

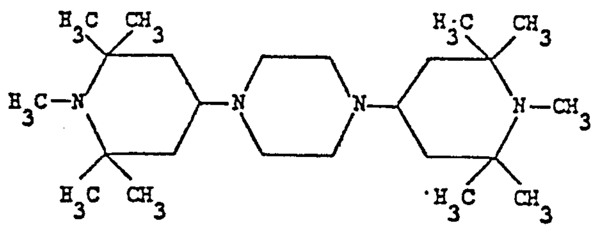

weisse Kristalle, Schmelzpunkt 210-12 °C.

Analyse für $C_{24}H_{48}N_4$
berechnet : C 73,41 % ; H 12,32 % ; N 14,27 %
gefunden : C 72,57 % ; H 12,16 % ; N 14,20 %.

Beispiel 3

Man erhitzt 202,8 g (0,6 Mol) N,N'-Bis-(2,2,6,6-tetramethyl-4-piperidyl)-aethylendiamin, 155,9 g (0,6 Mol) Aethyl-2,3-dibrompropionat, 165,8 g (1,2 Mol) wasserfreies Kaliumcarbonat und 1 000 ml Dimethylformamid 16 Stunden unter Rückfluss. Das Reaktionsgemisch wird heiss filtriert und das Filtrat zur Trockne gebracht.

Der erhaltene kristalline Rückstand wird aus n-Hexan umkristallisiert.

So erhält man die Verbindung der Formel

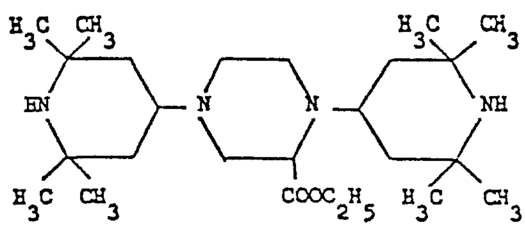

weisse Kristalle, Schmelzpunkt 101-2 °C.

Analyse für $C_{25}H_{48}N_4O_2$
berechnet : C 68,76 % ; H 11,08 % ; N 12,83 %
gefunden : C 68,12 % ; H 10,95 % ; N 12,65 %.

Beispiel 4

Man erhitzt 70,4 g (0,2 Mol) N,N'-Bis-(2,2,6,6-tetramethyl-4-piperidyl)-trimethylendiamin, 41,3 g (0,22 Mol) 1,2-Dibromäthan, 55,3 g (0,4 Mol) wasserfreies Kaliumcarbonat und 500 ml Dimethylformamid 20 Stunden unter Rückfluss.

Man filtriert heiss, bringt das Filtrat zur Trockne und kristallisiert den erhaltenen kristallinen Rückstand aus n-Hexan um.

So erhält man die Verbindung der Formel

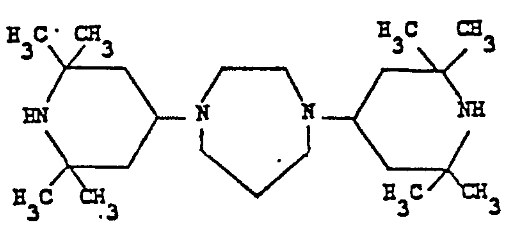

weisse Kristalle, Schmelzpunkt 86-7 °C.

Analyse für $C_{23}H_{46}N_4$
berechnet : C 72,96 % ; H 12,24 % ; N 14,80 %
gefunden : C 72,71 % ; H 12,15 % ; N 14,62 %.

Beispiel 5

Unter Entfernung des während der Reaktion freigesetzten Methanols erhitzt man 67,6 g (0,2 Mol) N, N'-Bis-(2,2,6,6-tetramethyl-4-piperidyl)-äthylendiamin und 23,6 g (0,2 Mol) Dimethyloxalat 3 Stunden auf 200 °C.
Das erhaltene Produkt wird aus Chloroform umkristallisiert.
So erhält man die Verbindung der Formel

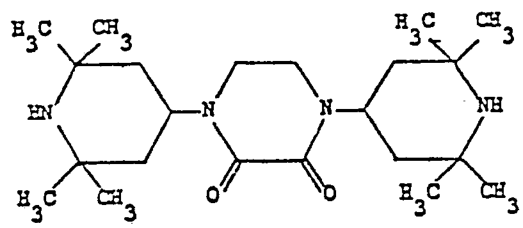

weise Kristalle vom Schmelzpunkt > 300 °C.

Analyse für $C_{22}H_{40}N_4O_2$
berechnet : C 67,31 % ; H 10,27 % ; N 14,27 %
gefunden : C 66,47 % ; H 10,12 % ; N 14,16 %.

Beispiel 6

Man erhitzt 135,2 g (0,4 Mol) N,N'-Bis-(2,2,6,6-tetramethyl-4-piperidyl)-äthylendiamin, 49,7 g (0, 4 Mol) Chloracetylchlorid und 800 ml Xylol 2 Stunden auf 80 °C.
Dann versetzt man mit einer Lösung von 33,6 g (0,84 Mol) Natriumhydroxyd in 100 ml Wasser und erhitzt 8 Stunden unter Rückfluss. Nach Abtrennung der wässrigen Phase bringt man die Xylolschicht zur Trockne und kristallisiert den erhaltenen Rückstand aus n-Octanum.
So erhält man die Verbindung der Formel

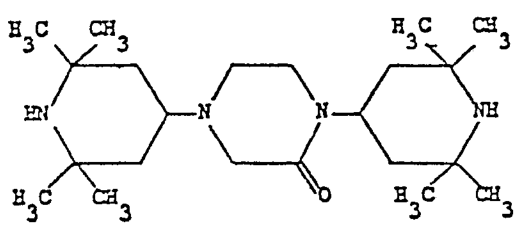

weisse Kristalle, Schmelzpunkt 177-8 °C.

Analyse für $C_{22}H_{42}N_4O$
berechnet : C 69,79 % ; H 11,18 % ; N 14,80 %
gefunden : C 68,95 % ; H 10,91 % ; N 14,64 %

Beispiel 7

Man erhitzt 135,2 g (0,4 Mol) N,N'-Bis-(2,2,6,6-tetramethyl-4-piperidyl)-äthylendiamin, 37,84 g (0,44 Mol) Methylacrylat und 100 ml Methanol 6 Stunden unter Rückfluss, entfernt dann das Lösungsmittel und erhitzt 16 Stunden auf 200 °C.
Das erhaltene Produkt wird aus Isopropanol umkristallisiert.
So erhält man die Verbindung der Formel

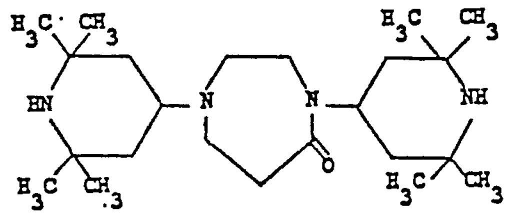

weisse Kristalle, Schmelzpunkt 203-5 °C.

Analyse für $C_{23}H_{44}N_4O$
berechnet : C 70,36 % ; H 11,29 % ; N 14,27 %
gefunden : C 69,75 % ; H 11,17 % ; N 14,13 %.

Beispiel 8

Unter Entfernung der während der Reaktion freigesetzten Methanols erhitzt man 33,8 g (0,1 Mol) N, N'-Bis-(2,2,6,6-tetramethyl-4-piperidyl)-äthylendiamin und 14,5 g (0,11 Mol) Dimethylmalonat 3 Stunden auf 170-180 °C.

Das erhaltene Produkt wird aus Isopropanol umkristallisiert.

So erhält man die Verbindung der Formel

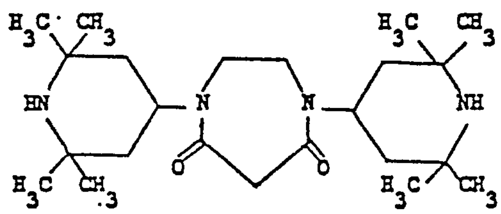

weisse Kristalle vom Schmelzpunkt > 300 °C.

Analyse für $C_{23}H_{42}N_4O_2$
berechnet : C 67,94 % ; H 10,41 % ; N 13,78 %
gefunden : C 67,38 % ; H 10,29 % ; N 13,73 %.

Beispiel 9

Unter Entfernung des während der Reaktion freigesetzten Methanols erhitzt man 35,2 g (0,1 Mol) N, N'-Bis-(2,2,6,6-tetramethyl-4-piperidyl)-1,2-diaminopropan und 14,5 g (0,11 Mol) Dimethylmalonat 3 Stunden auf 170-180 °C.

Das erhaltene Produkt wird aus Isopropanol umkristallisiert.

So erhält man die Verbindung der Formel

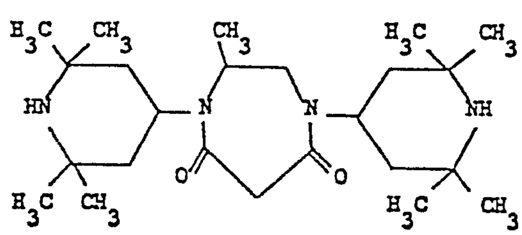

weisse Kristalle vom Schmelzpunkt > 300 °C.

Analyse für $C_{24}H_{44}N_4O_2$
berechnet : C 68,53 % ; H 10,54 % ; N 13,32 %
gefunden : C 67,75 % ; H 10,39 % ; N 13,29 %.

Beispiel 10

Unter Entfernung des während der Reaktion freigesetzten Aethanols erhitzt man 135,2 g (0,4 Mol) N, N'-Bis-(2,2,6,6-tetramethyl-4-piperidyl)-äthylendiamin und 95 g (0,44 Mol) Diäthyl-n-butylmalonat

30 Stunden auf 200-210 °C. Das erhaltene Produkt wird aus Isopropanol umkristallisiert.

So erhält man die Verbindung der Formel

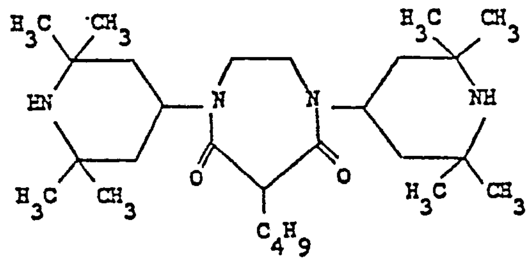

weisse Kristalle, Schmelzpunkt 215-7 °C.

Analyse für $C_{27}H_{50}N_4O_2$
berechnet : C 70,08 % ; H 10,89 % ; N 12,11 %
gefunden : C 69,06 % ; H 11.06 % ; N 11,94 %.

Beispiel 11

Man erhitzt 67,6 (0,2 Mol) N,N'-Bis-(2,2,6,6-tetramethyl-4-piperidyl)-äthylendiamin, 12 g (0,4 Mol) Paraformaldehyd, 15 g (0,2 Mol) Nitroäthan und 200 ml Methanol 12 Stunden auf 50-60 °C. Das erhaltene Gemisch wird zur Trockne gebracht und aus n-Hexan umkristallisiert.

So erhält man die Verbindung der Formel

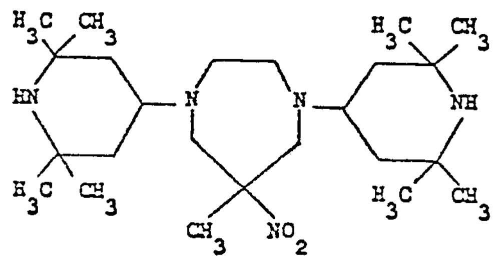

weisse Kristalle, Schmelzpunkt 147-9 °C.

Analyse für $C_{24}H_{47}N_5O_2$
berechnet : C 65,86 % ; H 10,82 % ; N 16,00 %
gefunden : C 65,40 % ; H 11,17 % ; N 15,74 %.

Beispiel 12

Man lässt 81,2 g (0,2 Mol) gemäss Beispiel 8 hergestelltes 1,4-Bis-(2,2,6,6-tetramethyl-4-piperidyl)-5,7-homopiperazindion, 36 g (1,2 Mol) Paraformaldehyd und 500 ml Methanol unter zeitweiligen Umrühren 2 Tage bei gewöhnlicher Temperatur stehen ; anschliessend wird das Gemisch unter 10 Atmosphären Druck in Gegenwart von 5 g 10 %iger Palladiumkohle bei 50-60 °C hydriert.

Nach Abtrennung des Katalysators entfernt man das Lösungsmittel durch Destillation und kristallisiert den Rückstand aus isopropanolum.

So erhält man die Verbindung der Formel

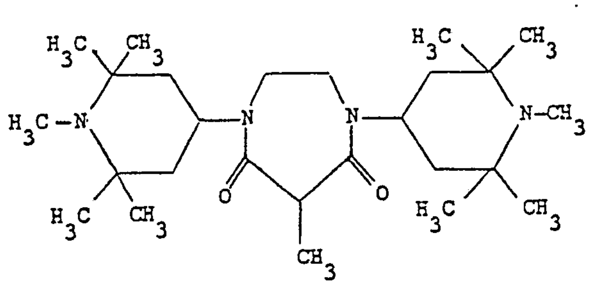

weisse Kristalle, Schmelzpunkt 232-3 °C.

Analyse für $C_{26}H_{48}N_4O_2$
berechnet : C 69,60 % ; H 10,78 % ; N 12,49 %
gefunden : C 69,86 % ; H 10,72 % ; N 12,21 %.

Beispiel 13

Unter Entfernung des während der Reaktion freigesetzten Methanols erhitzt man 67,6 g (0,2 Mol) N, N'-Bis-(2,2,6,6-tetramethyl-4-piperidyl)-äthylendiamin und 37,6 g (0,2 Mol) Dimethyl-isobutylmalonat 16 Stunden auf 160-170 °C und dann 2 Stunden auf 220 °C.

Man giesst das Reaktionsgemisch in Wasser und trennt den erhaltenen Niederschlag durch Filtrieren ab, wäscht mit Wasser, trocknet und kristallisiert dann aus Acetonum.

So erhält man die Verbindung der Formel

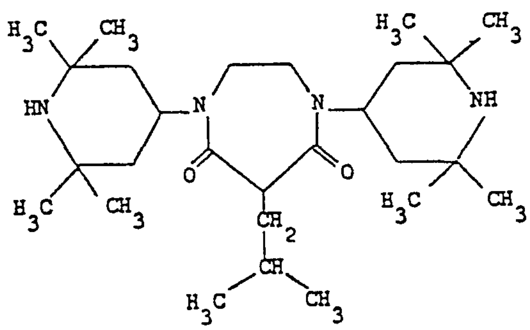

weisse Kristalle, Schmelzpunkt 221-2 °C.

Analyse für $C_{27}H_{50}N_4O_2$
berechnet : C 70,08 % ; H 10,89 % ; N 12,11 %
gefunden : C 69,56 % ; H 10,77 % ; N 11,74 %.

Beispiel 14

Man gibt 2,3 g (0,1 Grammatom) Natrium zu einem Gemisch aus 40,6 g (0,1 Mol) gemäss Beispiel 8 hergestelltem 1,4-Bis-(2,2,6,6-tetramethyl-4-piperidyl)-5,7-homopiperazindion, 100 ml Xylol und 100 ml Methanol. Man rührt bei Raumtemperatur bis zur völligen Umsetzung des Natriums und entfernt dann das Methanol durch Destillation unter Erhitzen bis auf 140 °C. Man kühlt auf 80 °C ab, versetzt mit 29 g (0,15 Mol) n-Octylbromid und erhitzt dann 22 Stunden unter Rückfluss. Das Reaktionsgemisch wird heiss filtriert und das Filtrat auf Raumtemperatur abgekühlt ; der erhaltene kristalline Niederschlag wird durch Filtrieren abgetrennt und zweimal aus n-Octan umkristallisiert.

Man erhält die Verbindung der Formel

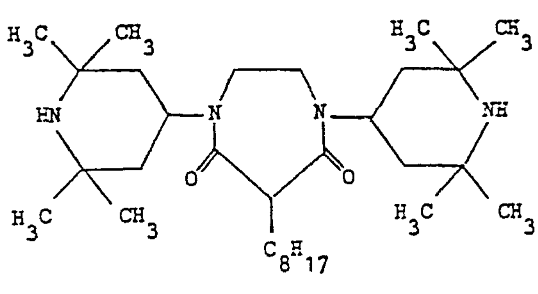

weisse Kristalle, Schmelzpunkt 141-2 °C.

Analyse für $C_{31}H_{58}N_4O_2$
berechnet : C 71,76 % ; H 11,27 % ; N 10,80 %
gefunden : C 70,63 % ; H 11,23 % ; N 10,35 %.

Beispiel 15

Man erhitzt 40,6 g (0,1 Mol) gemäss Beispiel 8 hergestelltes 1,4-Bis-(2,2,6,6-tetramethyl-4-piperidyl)-5,7-homopiperazindion, 36,7 g (0,1 Mol) 3,5-Di-t-butyl-4-hydroxybenzyl-diäthyldithiocarbamat und 500 ml Isopropanol auf 70 °C, versetzt im Verlauf von 15 Minuten mit einer Lösung von 4 g NaOH in 40 ml Wasser

und erhitzt 4 Stunden unter Rückfluss. Nach Abkühlen auf 50 °C gibt man eine Lösung von 12 g Essigsäure in 120 ml Wasser dazu, bringt dann das Reaktionsgemisch zur Trockne und kristallisiert den Rückstand zweimal aus Toluolum.

So erhält man die Verbindung der Formel

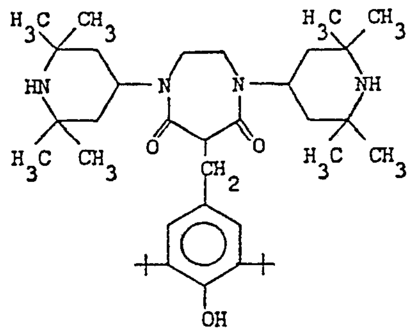

weisse Kristalle, Schmelzpunkt 247-9 °C.

Analyse für $C_{38}H_{64}N_4O_3$
berechnet : C 73,03 % ; H 10,32 % ; N 9,87 %
gefunden : C 73,09 % ; H 10.20 % ; N 9,71 %.

Beispiel 16

Man erhitzt 36,4 g (0,1 Mol) gemäss Beispiel 1 hergestelltes N,N'-Bis-(2,2,6,6-tetramethyl-4-piperidyl)-piperazin, 30,6 g (0,3 Mol) Essigsäureanhydrid, 60,6 g (0,6 Mol) Triäthylamin und 100 ml Toluol 15 Stunden unter Rückfluss.

Nach Abkühlung versetzt man mit 100 ml Wasser, rührt 15 Minuten, trennt dann den kristallinen Rückstand durch Filtrieren ab und wäscht diesen mit Wasser, trocknet und kristallisiert aus Propanolum.

So erhält man die Verbindung der Formel

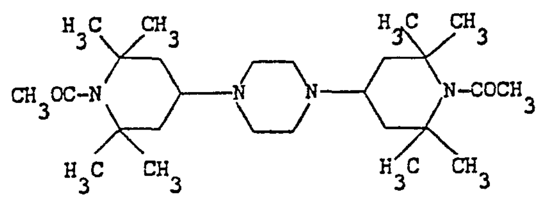

weisse Kristalle, Schmelzpunkt 181-2 °C.

Analyse für $C_{26}H_{48}N_4O_2$
berechnet : C 69,60 % ; H 10,78 % ; N 12,49 %
gefunden : C 69,36 % ; H 10,86 % ; N 12,37 %.

Wie anfangs erwähnt, sind die Verbindungen der Formel I sehr wirksam bei der Verbesserung der Licht-, Wärme- und Oxidationsbeständigkeit von synthetischen Polymeren wie beispielsweise Polyäthylen hoher und niedriger Dichte, Polypropylen, Aethylen/Propylencopolymeren, Aethylen/Vinylacetatcopolymeren, Polybutadien, Polyisopren, Polystyrol, Budadien/Styrolcopolymeren, Acrylnitril/Butadien/Styrolcopolymeren, Polymeren und Copolymeren von Vinylchlorid und Vinylidenchlorid, Polyoxymethylen, Polyurethanen, gesättigten und ungesättigten Polyestern, Polyamiden, Polycarbonaten, Polyacrylaten, Alkydharzen sowie Epoxidharzen.

Die Verbindungen der Formel I können im Gemisch mit synthetischen Polymeren in verschiedenen Anteilen eingesetzt werden, die von der Art des Polymeren, dem Endzweck und der Gegenwart weiterer Zusatzstoffe abhängen. Im allgemeinen ist ein Zusatz von 0,01 bis 5 Gew.-% Verbindungen der Formel I, bezogen auf das Gewicht der Polymeren, vorzugsweise von 0,1 bis 1 %, zweckmässig.

Die Verbindungen der Formel I können in polymeren Materialien nach verschiedenen Verfahren eingearbeitet werden, wie durch trockenes Einmischen in Pulverform oder nasses Einmischen in Form einer Lösung oder Suspension oder auch in Form eines Vorgemischs : das Polymer lässt sich dabei in Form von Pulver, Granulat, Lösung, Suspension oder Emulsion einsetzen. Die mit den Produkten der Formel I stabilisierten Polymeren lassen sich zur Herstellung von gestanzten Artikeln, Filmen, Bändern, Fasern, Endlosfäden und Lacken verwenden.

Ferner können sämtliche Mischungen von Verbindungen der Formel I mit synthetischen Polymeren gegebenenfalls weitere Zusatzstoffe enthalten, wie Antioxydantien, UV-absorbierende Stoffe, Nickelstabilisatoren, Pigmente, Füllstoffe, Weichmacher, Antistatika, Flammschutzmittel, Schmierstoffe, Korrosionshemmstoffe sowie Metalldesaktivatoren. Beispiele für in Mischung mit Verbindungen der Formel I verwendbare Zusatzstoffe sind insbesondere :

Phenolische Antioxydantien, wie beispielsweise 2,6-Di-t-butyl-p-kresol, 4,4'-Thio-bis-(3-methyl-6-butylphenol), 1,1,3-Tris-(2-methyl-4-hydroxy-5-t-butylphenyl)-butan, Octadecyl-3-(3,5-di-t-butyl-4-hydroxyphenyl)-propionat, Pentaerythrit-tetra-(3,5-di-t-butyl-4-hydroxyphenyl)-propionat, Tris-(3,5-di-t-butyl-4-hydroxybenzyl)-isocyanurat und Tris-(4-t-butyl-3-hydroxy-2,6-dimethylbenzyl)-isocyanurat ;

Sekundäre Antioxydantien, wie : Ester der Thiodipropionsäure wie beispielsweise Di-n-dodecylthiodipropionat und Di-n-octadecyl-thiodipropionat ; Aliphatische Sulfide und Disulfide, wie beispielsweise Di-n-dodecylsulfid, Di-n-octadecylsulfid und Di-n-octadecyldisulfid ; aliphatische, aromatische oder aliphatisch-aromatische Phosphite und Thiophosphite wie beispielsweise Tri-n-dodecylphosphit, Tris-(nonylphenyl)-phosphit, Tri-n-dodecyl-trithiophosphit, Phenyl-di-n-decylphosphit, Di-n-octadecyl-pentaerythrit-diphosphit, Tris-(2,4-di-t-butylphenyl)-phosphit und Tetrakis-(2,4-di-t-butylphenyl)-4,4'-biphenylendiphosphonit ;

UV-Absorbierende Stoffe wie beispielsweise 2-Hydroxy-4-n-octoxybenzophenon, 2-Hydroxy-4-n-dodecoxybenzophenon, 2-(2-Hydroxy-3,5-di-t-butylphenyl)-5-chlorbenztriazol, 2-(2-Hydroxy-3,5-di-t-amylphenyl)-benztriazol, 2,4-Di-t-butylphenyl-3,5-di-t-butyl-4-hydroxybenzoat, Phenylsalicylat, p-t-Butylphenyl-salicylat, 2,2'-Di-n-octoxy-5,5'-di-t-butyloxanilid und 2-Aethoxy-5-t-butyl-2'-äthoxyoxanilid ;

Lichtstabilisatoren auf Nickelbasis wie beispielsweise Ni-monoäthyl-3,5-di-t-butyl-4-hydroxybenzylphosphonat, der Butylaminkomplex des Ni-2,2'-thio-bis-(4-t-octylphenolats), Ni-2,2'-thio-bis-(4-t-octylphenylphenolat), Ni-dibutyldithiocarbamat, Ni-3,5-di-t-butyl-4-hydroxybenzoat und der Ni-komplex des 2-Hydroxy-4-n-octoxy-benzophenons ;

Organozinnstabilisatoren wie beispielsweise Dibutylzinn-maleinat, Dibutylzinn-laurat und Dioctylzinn-maleinat ;

Acrylester wie beispielsweise Aethyl-α-cyan-β, β-diphenylacrylat und Methyl-α-cyan-β-methyl-4-methoxycinnamat ;

Metallsalze höherer Fettsäuren wie beispielsweise Calcium-, Barium-, Cadmium-, Zink-, Blei- und Nickelstearat sowie Calcium-, Cadmium-, Zink- und Bariumlaurat ;

Organische und inorganische Pigmente wie beispielsweise Color Index Pigment Yellow 37, Color Index Pigment Yellow 83, Color Index Pigment Red 144, Color Index Pigment Red 48 : 3, Color Index Pigment Blue 15, Color Index Pigment Green 7, Titandioxyd und Eisenoxyd.

Die Wirksamkeit der erfindungsgemäss hergestellten Produkte als Stabilisatoren wird in den nachfolgenden Beispielen veranschaulicht, wo die in den Herstellungsbeispielen erhaltenen Produkte in einer synthetischen Polymermasse eingesetzt werden.

## Beispiel 17

Je 2 g der in Tabelle 1 angeführten Verbindungen und 1 g 2,6-Di-t-butyl-p-kresol (Antioxydans) werden mit 1 000 g Polypropylen MI = 3,2 (Moplen C, Produkt der Società Montedison) und 1 g Calciumstearat innig vermischt.

Das erhaltene Gemisch wird dann bei einer Temperatur von 200-230 °C extrudiert und zu Granulat verarbeitet, aus dem man Bänder einer Dicke von 40 μm und einer Breite von 3 mm herstellt. Die Arbeitsbedingungen sind :

Extrudertemperatur : 230-240 °C
Kopftemperatur : 240 °C
Streckverhältnis : 1 : 6

Die erhaltenen Bänder werden in einem Weather-Ometer 65 WR (ASTM G27-70) bei einer Temperatur der schwarzen Tafel von 63 °C ausgesetzt. Von Zeit zu Zeit werden Proben entnommen, an denen man die verbleibende Zugfestigkeit mittels eines Dynamometers mit konstanter Geschwindigkeit misst ; danach bestimmt man die zum Halbieren der ursprünglichen Zugfestigkeit erforderliche Aussetzungszeit ($T_{50}$).

Die erhaltenen Ergebnisse sind in Tabelle 1 angeführt.

Tabelle 1

| Stabilisator | $T_{50}$ (Stunden) |
|---|---|
| Verbindung aus Beispiel 1 | 2 180 |
| Verbindung aus Beispiel 2 | 2 090 |
| Verbindung aus Beispiel 3 | 1 850 |
| Verbindung aus Beispiel 4 | 1 970 |

| | |
|---|---|
| Verbindung aus Beispiel 6 | 1 330 |
| Verbindung aus Beispiel 10 | 1 450 |

Beispiel 18

Je 2 g der in Tabelle 2 angeführten Verbindungen werden mit 1 000 g Polyäthylen hoher Dichte, MI = 0,32 (Moplen RO ZB-5000, Produkt der Società Montedison) 0,5 g n-Octadecyl-3-(3,5-di-t-butyl-4-hydroxyphenyl)-propionat (Antioxydans) und 1 g Calciumstearat innig vermischt.

Das erhaltene Gemisch wird dann bei einer Temperatur von 190 °C extrudiert und zu Granulat verarbeitet, aus dem man mittels Formpressen bei 200 °C Scheiben einer Dicke von 0,2 mm erhält. Die Scheiben werden in einem Weather-Ometer 65 WR bei einer Temperatur der schwarzen Tafel von 63 °C ausgesetzt, und von Zeit zu Zeit verfolgt man die Zunahme an Carbonylgruppen (Δ CO) unter Verwendung von nicht ausgesetzten Proben, um die Anfangsabsorption des Polymeren auszugleichen. Man berechnet die erforderliche Zeit (T 0,1), bis Δ CO % = 0,1 bei 5,85 µm erreicht wird. Zum Vergleich stellt man unter den gleichen Bedingungen Scheiben aus Polymer ohne Zusatz eines Lichtstabilisators her.

Die erhaltenen Ergebnisse sind in Tabelle 2 angeführt.

Tabelle 2

| Stabilisator | T 0,1 (Stunden) |
|---|---|
| Ohne Lichtstabilisator | 320 |
| Verbindung aus Beispiel 1 | > 8 000 |
| Verbindung aus Beispiel 2 | > 8 000 |
| Verbindung aus Beispiel 3 | > 7 000 |
| Verbindung aus Beispiel 4 | > 8 000 |
| Verbindung aus Beispiel 8 | > 4 000 |
| Verbindung aus Beispiel 10 | > 4 000 |

**Ansprüche**

1. Verbindungen der Formel I

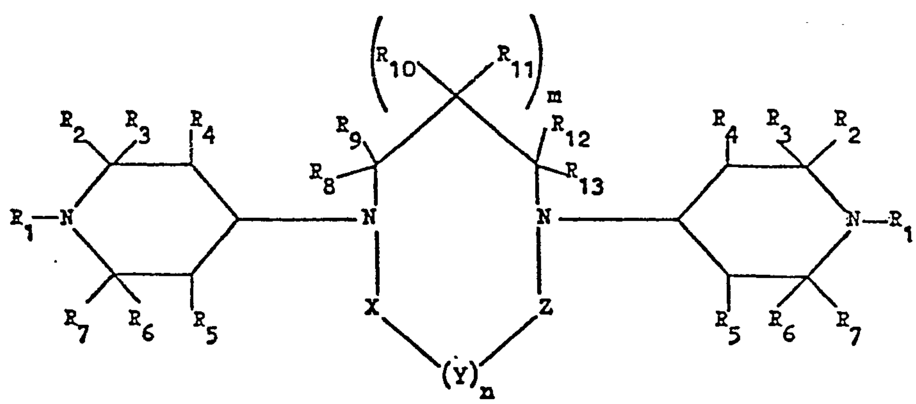

(I)

worin $R_1$ Wasserstoff, —O·, —CN, geradkettiges oder verzweigtes Alkyl mit 1 bis 20 Kohlenstoffatomen, Alkenyl oder Alkinyl mit 2 bis 20 Kohlenstoffatomen, gegebenenfalls durch $C_1$-$C_4$-Alkyl einfach bis dreifach substituiertes Benzyl oder Hydroxybenzyl, einen Rest $—COR_{14}$, $—COOR_{14}$, $—CH_2COOR_{14}$ oder $CONR_{14}R_{15}$, worin $R_{14}$ und $R_{15}$ gleich oder verschieden sein können und für geradkettiges oder verzweigtes $C_1$-$C_{20}$-Alkyl, $C_2$-$C_{20}$-Alkenyl, $C_5$-$C_{12}$-Cycloalkyl, gegebenenfalls durch $C_1$-$C_8$-Alkyl einfach bis dreifach substituiertes Phenyl, gegebenenfalls durch $C_1$-$C_4$-Alkyl einfach bis dreifach substituiertes Hydroxyphenyl, $C_7$-$C_{12}$-Aralkyl, 2,2,6,6-Tetramethyl-4-piperidyl, 1,2,2,6,6-Pentamethyl-4-piperidyl stehen oder, wenn sie an N gebunden sind, Wasserstoff sein oder zusammen mit dem N, an das sie gebunden sind, einen stickstoffhaltigen 5- bis 7-gliedrigen heterocyclischen Ring bilden können, einen Rest $—CH_2—CH(R_{16})—R_{17}$, worin $R_{16}$ für Wasserstoff oder Methyl und $R_{17}$ für —OH, $—OR_{14}$, $—OCOR_{14}$ oder

—$OCONR_{14}R_{15}$ stehen, worin $R_{14}$ und $R_{15}$ die oben angegebene Bedeutung haben, oder einen Rest

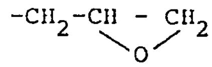

darstellt, $R_2$, $R_3$, $R_6$ und $R_7$ gleich oder verschieden sein können und Alkyl mit 1 bis 6 C-Atomen bedeuten, $R_4$ und $R_5$ gleich oder verschieden sein können und für Wasserstoff oder Alkyl mit 1 bis 6 C-Atomen stehen, $R_8$, $R_9$, $R_{10}$, $R_{11}$, $R_{12}$ und $R_{13}$ gleich oder verschieden sein können und Wasserstoff oder Alkyl mit 1 bis 6 C-Atomen darstellen, m und n null oder 1 sind, X und Z gleich oder verschieden sein können und

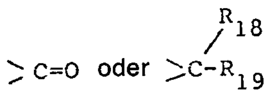
oder

bedeuten, worin $R_{18}$ für Wasserstoff oder $C_1$-$C_{20}$-Alkyl und $R_{19}$ für Wasserstoff, $C_1$-$C_{20}$-Alkyl oder einen Rest —$(CH_2)_r$—$COOR_{14}$ oder —$CONR_{14}R_{15}$ stehen, wobei $R_{14}$ und $R_{15}$ die oben angegebene Bedeutung haben und r eine ganze Zahl von 0 bis 10 ist, sowie

Y 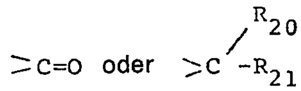
oder

darstellt, worin $R_{20}$ für Wasserstoff oder $C_1$-$C_{20}$-Alkyl und $R_{21}$ für Wasserstoff, $C_1$-$C_{20}$-Alkyl, gegebenenfalls durch $C_1$-$C_4$-Alkyl einfach bis dreifach substituiertes Benzyl oder Hydroxybenzyl, 2,2,6,6-Tetramethyl-4-piperidyl, 1,2,2,6,6-Pentamethyl-4-piperidyl oder einen Rest —OH, —$NO_2$, —$NR_{22}R_{23}$ oder —NH—$COR_{24}$ stehen, wobei $R_{22}$ und $R_{23}$ gleich oder verschieden sein können und Wasserstoff, $C_1$-$C_{20}$-Alkyl, Benzyl oder gegebenenfalls durch $C_1$-$C_4$-Alkyl einfach bis dreifach substituiertes Hydroxybenzyl sowie $R_{24}$ $C_1$-$C_{20}$-Alkyl, gegebenenfalls durch $C_1$-$C_8$-Alkyl einfach bis dreifach substituiertes Phenyl oder durch $C_1$-$C_4$-Alkyl einfach bis dreifach substituiertes Hydroxyphenyl bedeuten.

2. Verbindungen nach Anspruch 1 der Formel I, dadurch gekennzeichnet, dass $R_1$ Wasserstoff oder $C_1$-$C_6$-Alkyl darstellt, $R_2$, $R_3$, $R_6$ und $R_7$ Methyl oder Aethyl bedeuten, $R_4$, $R_5$, $R_8$, $R_9$, $R_{10}$, $R_{11}$, $R_{12}$ und $R_{13}$ für Wasserstoff oder Methyl stehen,

X und Z 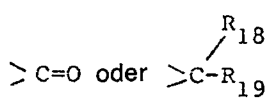
oder

darstellen, wobei $R_{18}$ vorzugsweise Wasserstoff oder Methyl sowie $R_{19}$ Wasserstoff oder Aethoxycarbonyl bedeuten,

Y 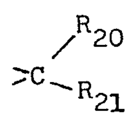

darstellt, worin $R_{20}$ vorzugsweise für Wasserstoff oder $C_1$-$C_{12}$-Alkyl und $R_{21}$ für Wasserstoff, $C_1$-$C_{12}$-Alkyl, Benzyl, 3,5-Di-t-butyl-4-hydroxybenzyl, —OH, —$NO_2$ oder —$NH_2$ stehen, sowie m und n null oder 1 sind.

3. Verbindungen nach Anspruch 1 der Formel I, dadurch gekennzeichnet, dass $R_2$ und $R_6$ für Aethyl, $R_3$, $R_4$ und $R_7$ für Methyl sowie $R_5$ für Wasserstoff stehen.

4. Verbindungen nach Anspruch 1 der Formel I, dadurch gekennzeichnet, dass $R_2$, $R_3$, $R_6$ und $R_7$ für Methyl sowie $R_4$ und $R_5$ für Wasserstoff stehen.

5. Verfahren zur Herstellung von Verbindungen der Formel I nach Anspruch 1, dadurch gekennzeichnet, dass man ein N,N'-Bis-(polyalkyl-4-piperidyl)-alkylendiamin der Formel II

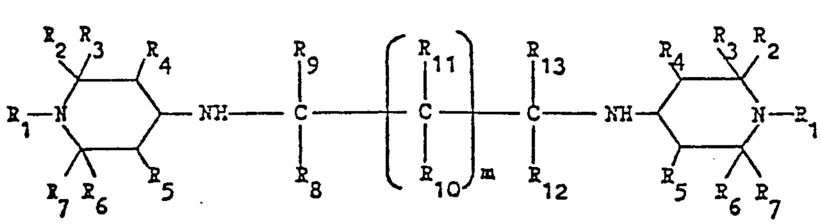
(II)

worin $R_1$, $R_2$, $R_3$, $R_4$, $R_5$, $R_6$, $R_7$, $R_8$, $R_9$, $R_{10}$, $R_{11}$, $R_{12}$ und $R_{13}$ die oben angegebene Bedeutung haben,

a) durch Umsetzung mit Verbindungen der Formel III

$$A—X—(Y)_n—Z—B \quad \text{(III)}$$

worin X, Y, Z und n die oben angegebene Bedeutung haben sowie A und B ein Halogen, Chlor oder Brom, bzw., falls X und Z $>C = O$ sind, eine Gruppe $—OR_{25}$ darstellen, wobei $R_{25}$ für $C_1$-$C_4$-Alkyl, Phenyl oder Methylphenyl steht, cyclisiert,

b) durch Umsetzung mit Verbindungen der Formel IV

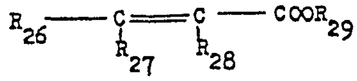

(IV)

worin $R_{26}$, $R_{27}$ und $R_{28}$ gleich oder verschieden sind und für Wasserstoff oder $C_1$-$C_4$-Alkyl und $R_{29}$ für $C_1$-$C_4$-Alkyl, Phenyl oder Methylphenyl stehen, cyclisiert oder

c) durch Umsetzung mit Formaldehyd und einer aliphatischen Nitroverbindung der Formel V

$$R_{20}—CH_2—NO_2 \quad \text{(V)}$$

worin $R_{20}$ die zuvor angegebene Bedeutung hat, cyclisiert und gegebenenfalls die so erhaltenen Nitroverbindungen nach herkömmlichen Reduktionsverfahren zu Aminoverbindungen reduziert.

6. Verfahren nach Anspruch 5, dadurch gekennzeichnet, dass man Verbindungen der Formel I, worin $R_1$ H ist,

a) mit Verbindungen der Formel $E—R_1$ umsetzt, worin E Halogen, vorzugsweise Cl, Br oder J, und $R_1$ CN, Alkyl, Alkenyl, Alkinyl obiger Bedeutung, Benzyl, $—COR_{14}$, $—COOR_{14}$, $—CH_2—COOR_{14}$ oder $—CONR_{14}R_{15}$ darstellen, wobei $R_{14}$ und $R_{15}$ die oben angegebene Bedeutung haben,

b) mit Ameisensäure und Formaldehyd umsetzt, um Verbindungen der Formel I zu erhalten, worin $R_1$ $CH_3$ ist,

c) mit Wasserstoffperoxyd in Gegenwart von Natriumwolframat oder mit einer Persäure zu Verbindungen der Formel I, in denen $R_1$ für —O· steht, umsetzt,

d) mit einem Alkylenoxyd zu Verbindungen der Formel I umsetzt, in denen $R_1$ für 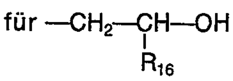

steht und $R_{16}$ die oben angegebene Bedeutung hat, und die so erhaltenen Verbindungen gegebenenfalls mit einer Verbindung der Formel $E—R_{14}$, $E—COR_{14}$ oder $E—CONR_{14}R_{15}$, worin E, $R_{14}$ und $R_{15}$ die oben angegebene Bedeutung haben, umsetzt, oder

e) mit Epichlorhydrin und Aetzalkali zu Verbindungen der Formel I umsetzt, worin

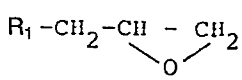

darstellt.

7. Gegen Licht, Wärme und Oxidation stabilisierte polymere Zusammensetzungen, dadurch gekennzeichnet, dass sie ein synthetisches Polymer und eine Stabilisatorverbindung der Formel I nach Anspruch 1 in einer Menge von 0,01 bis 5 Gew.-%, vorzugsweise von 0,1 bis 1 Gew.-%, bezogen auf das Gewicht des synthetischen Polymeren, enthalten.

8. Zusammensetzungen nach Anspruch 7, dadurch gekennzeichnet, dass sie zusätzlich zu den erfindungsgemäßen Stabilisatoren ferner weitere übliche Zusatzstoffe für synthetische Polymere enthalten.

9. Zusammensetzungen nach Anspruch 7, dadurch gekennzeichnet, dass als synthetisches Polymer Polyäthylen oder Polypropylen vorliegt.

**Claims**

1. A compound of the formula

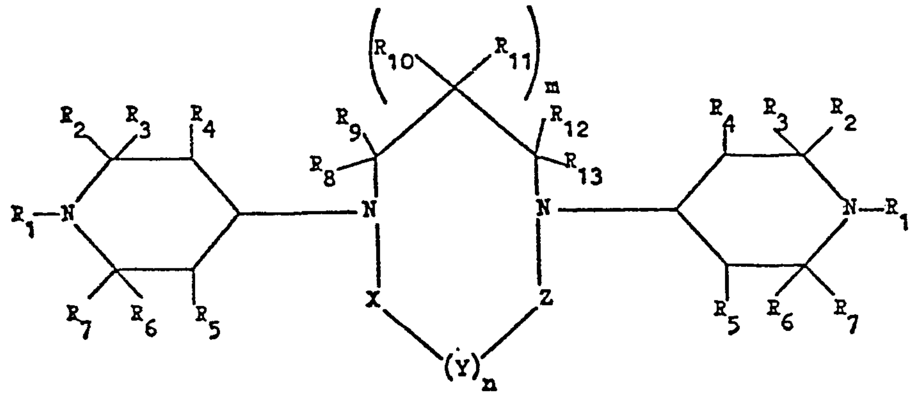

(I)

in which $R_1$ is hydrogen, —O·, —CN, a linear or branched alkyl radical containing from 1 to 20 carbon atoms, an alkenyl or alkynyl radical containing from 2 to 20 carbon atoms, benzyl which is unsubstituted or substituted by 1 to 3 $C_1$-$C_4$-alkyl radicals or hydroxybenzyl which is unsubstituted or substituted by 1 to 3 $C_1$-$C_4$-alkyl radicals ; or $R_1$ is a —$COR_{14}$, —$COOR_{14}$, —$CH_2COOR_{14}$ or —$CONR_{14}R_{15}$ radical, in which $R_{14}$ and $R_{15}$, which may be identical or different, are linear or branched $C_1$-$C_{20}$-alkyl, $C_2$-$C_{20}$-alkenyl, $C_5$-$C_{12}$-cycloalkyl, phenyl which is unsubstituted or substituted by 1 to 3 $C_1$-$C_8$-alkyl radicals, hydroxyphenyl which is unsubstituted or substituted by 1 to 3 $C_1$-$C_4$-alkyl radicals, $C_7$-$C_{12}$-aralkyl, 2,2,6,6-tetramethyl-4-piperidyl, 1,2,2,6,6-pentamethyl-4-piperidyl or, when they are attached to N, can be hydrogen or, together with the N to which they are attached, can form a nitrogen-containing heterocyclic ring with 5-7 members ; or $R_1$ is a —$CH_2$—CH—$R_{17}$ radical, in which $R_{16}$ is hydrogen or methyl and $R_{17}$ is —OH,
$R_{16}$

—$OR_{14}$, —$OCOR_{14}$ or —$OCONR_{14}R_{15}$, in which $R_{14}$ and $R_{15}$ are as defined above ; or $R_1$ is a

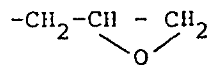

radical ;

$R_2$, $R_3$, $R_6$ and $R_7$, which may be identical or different, are an alkyl radical containing 1 to 6 carbon atoms ; $R_4$ and $R_5$, which may be identical or different, are hydrogen or an alkyl radical containing from 1 to 6 carbon atoms ; $R_8$, $R_9$, $R_{10}$, $R_{11}$, $R_{12}$ and $R_{13}$, which may be identical or different, are hydrogen or an alkyl radical containing 1 to 6 carbon atoms ; m and n are zero or 1 ; X and Z, which may be identical or different, are

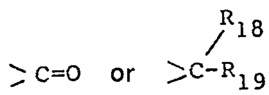

or

in which $R_{18}$ is hydrogen or $C_1$-$C_{20}$-alkyl and $R_{19}$ is hydrogen, $C_1$-$C_{20}$-alkyl or a —$(CH_2)_r$—$COOR_{14}$ radical, or a —$CONR_{14}R_{15}$ radical in which $R_{14}$ and $R_{15}$ are as defined above and r is an integer from 0 to 10 ; Y is

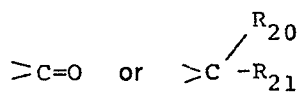

or

in which $R_{20}$ is hydrogen or $C_1$-$C_{20}$-alkyl and $R_{21}$ is hydrogen, $C_1$-$C_{20}$-alkyl, benzyl which is unsubstituted or substituted by 1 to 3 $C_1$-$C_4$-alkyl radicals, hydroxybenzyl which is unsubstituted or substituted by 1 to 3 $C_1$-$C_4$-alkyl radicals, 2,2,6,6-tetramethyl-4-piperidyl, 1,2,2,6,6-pentamethyl-4-piperidyl or a —OH, —$NO_2$, —$NR_{22}R_{23}$ or —NH—$COR_{24}$ radical, in which $R_{22}$ and $R_{23}$, which may be identical or different, are hydrogen, $C_1$-$C_{20}$-alkyl, benzyl or hydroxybenzyl substituted by 1 to 3 $C_1$-$C_4$-alkyl radicals and $R_{24}$ is $C_1$-$C_{20}$-alkyl, phenyl which is unsubstituted or substituted by 1 to 3 $C_1$-$C_8$-alkyl radicals or hydroxyphenyl which is substituted by 1 to 3 $C_1$-$C_4$-alkyl radicals.

2. A compound according to claim 1 of the formula I, in which $R_1$ is hydrogen or $C_1$-$C_6$-alkyl, $R_2$, $R_3$, $R_6$ and $R_7$ are methyl or ethyl, $R_4$, $R_5$, $R_8$, $R_9$, $R_{10}$, $R_{11}$, $R_{12}$ and $R_{13}$ are hydrogen or methyl, X and Z are

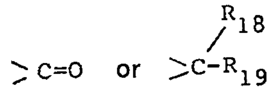

or

in which $R_{18}$ is preferably hydrogen or methyl and $R_{19}$ is hydrogen or ethoxycarbonyl, Y is

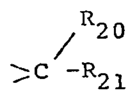

in which $R_{20}$ is preferably hydrogen or $C_1$-$C_{12}$-alkyl and $R_{21}$ is hydrogen, $C_1$-$C_{12}$-alkyl, benzyl, 3,5-di-t-butyl-4-hydroxybenzyl, —OH, —$NO_2$ or —$NH_2$, and m and n are zero or 1.

3. A compound according to claim 1 of the formula I, in which $R_2$ and $R_6$ are ethyl, $R_3$, $R_4$ and $R_7$ are methyl and $R_5$ is hydrogen.

4. A compound according to claim 1 of the formula I, in which $R_2$, $R_3$, $R_6$ and $R_7$ are methyl and $R_4$ and $R_5$ are hydrogen.

5. A process for the preparation of a compound of the formula I according to claim 1, wherein a N,N'-bis-(polyalkyl-4-piperidyl)-alkylenediamine of the formula

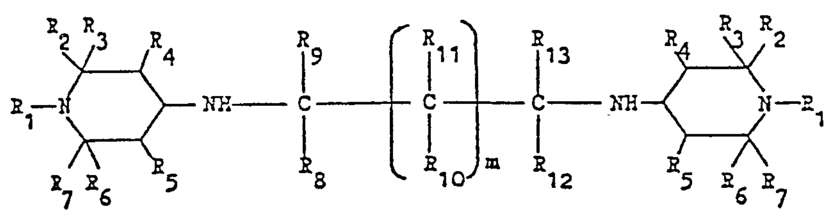

(II)

in which $R_1$, $R_2$, $R_3$, $R_4$, $R_5$, $R_6$, $R_7$, $R_8$, $R_9$, $R_{10}$, $R_{11}$, $R_{12}$ and $R_{13}$ are as defined above,

a) is cyclised by reaction with with a compound of the formula III

$$A—X—(Y)_n—Z—B \qquad (III)$$

in which X, Y, Z and n are as defined above and A and B are halogen, preferably chlorine or bromine, or, if X and Z are $=C=O$, A and B are a $—OR_{25}$ group, in which $R_{25}$ is $C_1$-$C_4$-alkyl, phenyl or methylphenyl, or

b) is cyclised by reaction with a compound of the formula IV

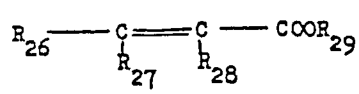

(IV)

in which $R_{26}$, $R_{27}$ and $R_{28}$, which may be identical or different, are hydrogen or $C_1$-$C_4$-alkyl and $R_{29}$ is $C_1$-$C_4$-alkyl, phenyl or methylphenyl, or

c) is cyclised by reaction with formaldehyde and an aliphatic nitro compound of the formula V

$$R_{20}—CH_2—NO_2 \qquad (V)$$

in which $R_{20}$ is as defined above, and the nitro compound so obtained is reduced, if desired, to the amino compound by a conventional reduction process.

6. A process according to claim 5, wherein a compound of the formula I, in which $R_1$ is H, is

a) reacted with a compound of the formula $E—R_1$, in which E is halogen, preferably Cl, Br or I, and $R_1$ is CN, alkyl, alkenyl, alkynyl, as defined above, benzyl, $—COR_{14}$, $—COOR_{14}$, $—CH_2—COOR_{14}$ or $—CONR_{14}R_{15}$ in which $R_{14}$ and $R_{15}$ are as defined above, or

b) is reacted with formic acid and formaldehyde to give a compound of the formula I in which $R_1$ is $CH_3$, or

c) is reacted with hydrogen peroxide in the presence of sodium tungstate or with a per-acid to give a compound of the formula I, in which $R_1$ is O, or

d) is reacted with an alkylene oxide to give a compound of the formula I, in which $R_1$ is $—CH_2—CH(R_{16})—OH$ and $R_{16}$ is as defined above, and the compound so obtained is reacted, if desired, with a compound of the formula $E—R_{14}$, $E—COR_{14}$ or $E—CONR_{14}R_{15}$, in which E, $R_{14}$ and $R_{15}$ are as defined above, or

e) is reacted with epichlorohydrin and an alkali metal hydroxide to give a compound of the formula I, in which $R_1$ is

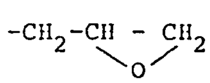

7. A light-stabilised, heat-stabilised and oxidation-stabilised polymer composition which comprises a synthetic polymer and a stabiliser of the formula I according to claim 1, in an amount of 0.01 to 5 %, preferably of 0.1 to 1 % by weight, based on the weight of the synthetic polymer.

8. A composition according to claim 7, which in addition to containing the stabiliser of the invention also contains other conventional additives for synthetic polymers.

9. A composition according to claim 7, wherein the synthetic polymer is polyethylene or polypropylene.

**Revendications**

1. Les composés de formule générale

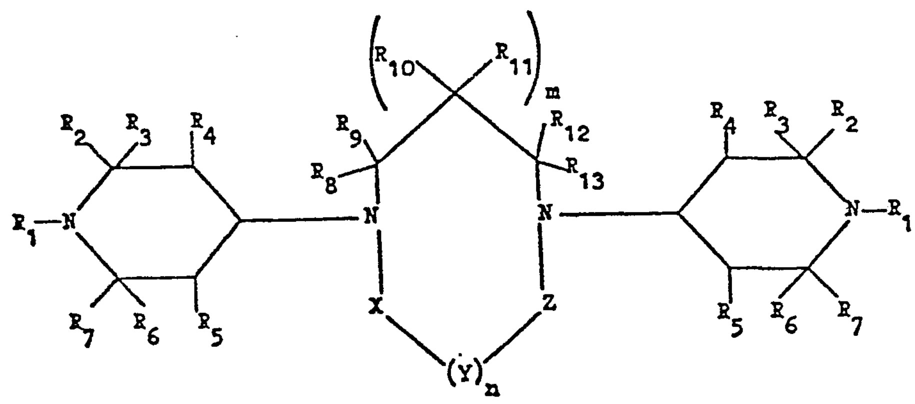

(I)

dans laquelle $R_1$ désigne l'hydrogène, —O·, —CN, un alkyle à chaine droite ou ramifiée en $C_1$ à $C_{20}$, un alcényle ou un alcynyle en $C_2$ à $C_{20}$, un benzyle ou un hydroxybenzyle portant éventuellement un à trois alkyles en $C_1$ à $C_4$, un radical $-COR_{14}$, $-COOR_{14}$, $-CH_2COOR_{14}$ ou $-CONR_{14}R_{15}$, $R_{14}$ et $R_{15}$, qui peuvent être identiques ou différents l'un de l'autre, étant chacun un alkyle en $C_1$-$C_{20}$ à chaine droite ou ramifiée, un alcényle en $C_2$-$C_{20}$, un cycloalkyle en $C_5$-$C_{12}$, un phényle éventuellement substitué par un à trois alkyles en $C_1$-$C_8$, un hydroxyphényle éventuellement substitué par un à trois alkyles en $C_1$-$C_4$, un aralkyle en $C_7$-$C_{12}$, un 2,2,6,6-tétraméthyl-4-pipéridyle ou un 1,2,2,6,6-pentaméthyl-4-pipéridyle, ou bien, s'ils sont liés à l'azote, peuvent être de l'hydrogène ou former ensemble et avec l'atome d'azote auquel ils sont liés un hétérocycle azoté pentagonal, hexagonal ou heptagonal, ou encore un radical $-CH_2-\underset{R_{18}}{\underset{|}{CH}}-R_{17}$, $R_{16}$ représentant l'hydrogène ou un méthyle et $R_{17}$ un groupe —OH, $-OR_{14}$, $-OCOR_{14}$ ou $-OCONR_{14}R_{15}$, $R_{14}$ et $R_{15}$ ayant les mêmes significations que ci-dessus, ou un radical

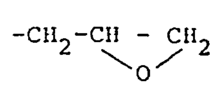

$R_2$, $R_3$, $R_6$ et $R_7$, qui peuvent être identiques ou différents les uns des autres, désignent des alkyles en $C_1$ à $C_8$, $R_4$ et $R_5$, identiques ou différents l'un de l'autre, désignent chacun l'hydrogène ou un alkyle en $C_1$ à $C_8$, $R_8$, $R_9$, $R_{10}$, $R_{11}$, $R_{12}$ et $R_{13}$, identiques ou différents l'un de l'autre, désignent chacun l'hydrogène ou un alkyle en $C_1$ à $C_8$, m et n sont chacun le nombre 0 ou 1, X et Z, identiques ou différents l'un de l'autre, représentent chacun un groupe

$>C=O$ ou 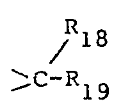

$R_{18}$ désignant l'hydrogène ou un alkyle en $C_1$-$C_{20}$ et $R_{19}$ l'hydrogène, un alkyle en $C_1$-$C_{20}$ ou un radical $-(CH_2)_r-COOR_{14}$ ou $-CONR_{14}R_{15}$, $R_{14}$ et $R_{15}$ ayant les significations précédemment données et r étant un entier de 0 à 10, et Y représente un groupe

$>C=O$ ou 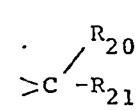

$R_{20}$ désignant l'hydrogène ou un alkyle en $C_1$-$C_{20}$ et $R_{21}$ l'hydrogène, un alkyle en $C_1$-$C_{20}$, un benzyle ou un hydroxybenzyle éventuellement substitué par un à trois alkyles en $C_1$-$C_4$, un 2,2,6,6-tétraméthyl-4-pipéridyle ou un 1,2,2,6,6-pentaméthyl-4-pipéridyle ou encore un radical —OH, —$NO_2$, —$NR_{22}R_{23}$ ou —NH—$COR_{24}$, $R_{22}$ et $R_{23}$, identiques ou différents l'un de l'autre, étant chacun l'hydrogène, un alkyle en $C_1$-$C_{20}$, un benzyle ou un hydroxybenzyle, éventuellement substitué par un à trois alkyles en $C_1$-$C_4$, et $R_{24}$ étant un alkyle en $C_1$-$C_{20}$, un phényle éventuellement substitué par un à trois alkyles en $C_1$-$C_8$ ou un hydroxyphényle éventuellement substitué par un à trois alkyles en $C_1$-$C_4$.

2. Composés de formule I selon la revendication 1 dans lesquels $R_1$ représente l'hydrogène ou un alkyle en $C_1$-$C_6$, $R_2$, $R_3$, $R_6$ et $R_7$ sont chacun un méthyle, $R_4$, $R_5$, $R_8$, $R_9$, $R_{10}$, $R_{11}$, $R_{12}$ et $R_{13}$ sont l'hydrogène ou un méthyle, X et z sont chacun un groupe

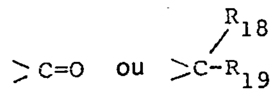

$R_{18}$ étant de préférence l'hydrogène ou un méthyle et $R_{19}$ l'hydrogène ou un éthoxycarbonyle, Y est un groupe

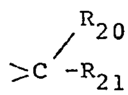

$R_{20}$ étant de préférence l'hydrogène ou un alkyle en $C_1$-$C_{12}$ et $R_{21}$ l'hydrogène, un alkyle en $C_1$-$C_{12}$, ou un groupe benzyle, 3,5-di-t-butyl-4-hydroxybenzyle, —OH, —$NO_2$ ou —$NH_2$, et m et n sont chacun le nombre 0 ou 1.

3. Composés de formule I selon la revendication 1 dans lesquels $R_2$ et $R_6$ sont chacun le groupe éthyle, $R_3$, $R_4$ et $R_7$ sont chacun le groupe méthyle et $R_5$ est l'hydrogène.

4. Composés de formule I selon la revendication 1 dans lesquels $R_2$, $R_3$, $R_6$, $R_7$ sont chacun le groupe méthyle et $R_4$ et $R_5$ sont chacun l'hydrogène.

5. Procédé de préparation des composés de formule I selon la revendication 1, caractérisé en ce qu'on cyclise une N,N'-bis-(polyalkyl-4-pipéridyl)-alkylène-diamine de formule II

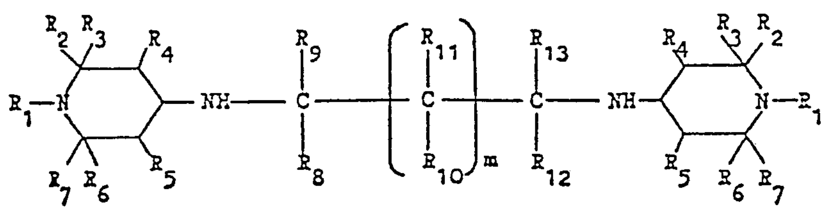

(II)

a) par réaction avec des composés de formule III

$$A—X—(Y)_n—Z—B \qquad (III)$$

(A et B désignant chacun un halogène, chlore ou brome, ou encore, si X et Z sont chacun le groupe $\geq C = O$, un groupe —$OR_{25}$, $R_{25}$ étant un alkyle en $C_1$-$C_4$, un phényle ou un méthylphényle), ou bien

b) par réaction avec des composés de formule IV

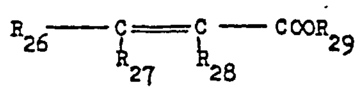

(IV)

($R_{26}$, $R_{27}$ et $R_{28}$, qui peuvent être identiques ou différents l'un de l'autre, désignant chacun l'hydrogène ou un alkyle en $C_1$-$C_4$ et $R_{29}$ un alkyle en $C_1$-$C_4$, un méthyle ou un méthyl-phényle), ou bien

c) par réaction avec le formaldéhyde et un composé nitré aliphatique de formule V

$$R_{20}—CH_2—NO_2 \qquad (V)$$

($R_{20}$ ayant la signification précédemment donnée), et dans ce dernier cas on réduit éventuellement le composé nitré ainsi formé en composé aminé par un procédé de réduction connu.

6. Procédé selon la revendication 5, caractérisé en ce que l'on fait réagir des composés de formule I dans lesquels $R_1$ est l'hydrogène,

a) avec des composés de formule E—$R_1$, E désignant un halogène, de préférence le chlore, le brome ou l'iode, et $R_1$ un groupe CN, un alkyle, un alcényle ou un alcynyle tel que ci-dessus, un benzyle ou

encore un groupe $-COR_{14}$, $-COOR_{14}$, $-CH_2COOR_{14}$ ou $-CONR_{14}R_{15}$, $R_{14}$ et $R_{15}$ ayant les significations précédemment données, ou bien

b) avec l'acide formique et le formaldéhyde pour obtenir des composés de formule I dans lesquels $R_1$ est le groupe $CH_3$, ou bien

c) avec le peroxyde d'hydrogène en présence de tungstate de sodium ou avec un peracide pour former des composés de formule I dans lesquels $R_1$ représente —O·, ou bien

d) avec un oxyde d'alkylène pour former les composés de formule I dans lesquels $R_1$ est un groupe $-CH_2-\underset{R_{16}}{\underset{|}{CH}}-OH$, $R_{16}$ ayant la signification précédente, puis le cas échéant l'on fait réagir les composés ainsi obtenus avec un composé de formule $E-R_{14}$, $E-COR_{14}$ ou $E-CONR_{14}R_{15}$, et, $R_{14}$ et $R_{15}$ ayant les significations précédentes, ou encore

e) avec l'épichlorhydrine et un hydroxyde métal alcalin pour former des composés de formule I dans lesquels $R_1$ est un groupe

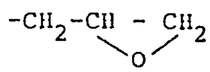

7. Compositions de matières polymères stabilisées contre l'action de la lumière, de la chaleur et de l'oxydation, caractérisées en ce qu'elles comprennent un polymère synthétique et un composé stabilisant de formule I selon la revendication 1 dans une proportion de 0,01 à 5 %, de préférence de 0,1 à 1 %, du poids du polymère synthétique.

8. Compositions selon la revendication 7 caractérisées en ce quelles comprennent en outre d'autres additifs usuels pour polymères synthétiques.

9. Compositions selon la revendication 7 ou 8, caractérisées en ce que le polymère synthétique est du polyéthylène ou du polypropylène.